# EUROPÄISCHE PATENTSCHRIFT

(11) **EP 2 006 688 B1**
(45) Veröffentlichungstag und Bekanntmachung des Hinweises auf die Patenterteilung: **27.07.2016**
(21) Anmeldenummer: 08164348.8
(22) Anmeldetag: 03.04.2002
(51) Int. Cl.: G01N 33/74, G01N 33/78

(54) **Bestimmung der wirksamen Parathormon-Aktivität in einer Probe**
Method of determining effective parathormone activity in a sample
Procédé de détermination de l'activité effective de la parathormone dans un échantillon

(30) Priorität: 03.04.2001 DE 10116552
(43) Veröffentlichungstag der Anmeldung: 24.12.2008
(62) Teilanmeldung aus: 02737914.8
(73) Patentinhaber: Immundiagnostik AG, 64625 Bensheim (DE)
(72) Erfinder: Armbruster, Franz Paul, 67240 Bobenheim-Roxheim (DE); Roth, Heinz-Jürgen, 69126 Heidelberg (DE); Schmidt-Gayk, Heinrich, deceased (DE)
(74) Vertreter: Benedum, Ulrich Max

(56) Entgegenhaltungen:
- EP-B1- 1 151 307
- WO-A-01/44818
- WO-A-96/10041
- WO-A1-00/42437
- MAEGERLEIN M ET AL: "A NEW IMMUNOENZYMOMETRIC ASSAY FOR BIOACTIVE N-TERMINAL HUMAN PARATHYROID HORMONE FRAGMENTS AND ITS APPLICATION IN PHARMACOKINETIC STUDIES IN DOGS" ARZNEIMITTEL FORSCHUNG. DRUG RESEARCH, EDITIO CANTOR. AULENDORF, DE, Bd. 48, Nr. 2, 1998, Seiten 199-204, XP001010446 ISSN: 0004-4172
- ZULL J E ET AL: "Effect of methionine oxidation and deletion of amino-terminal residues on the conformation of parathyroid hormone. Circular dichroism studies." THE JOURNAL OF BIOLOGICAL CHEMISTRY. UNITED STATES 5 APR 1990, Bd. 265, Nr. 10, 5. April 1990 (1990-04-05), Seiten 5671-5676, XP002222807 ISSN: 0021-9258
- LOGUE F C ET AL: "PRODUCTION AND CHARACTERISATION OF MONOCLONAL ANTIBODIES TO PARATHYROID HORMONE (1-34)" JOURNAL OF IMMUNOLOGICAL METHODS, ELSEVIER SCIENCE PUBLISHERS B.V.,AMSTERDAM, NL, Bd. 137, Nr. 2, 1991, Seiten 159-166, XP001010448 ISSN: 0022-1759
- REICHEL HELMUT ET AL: "Influence of PTH assay methodology on differential diagnosis of renal bone disease." NEPHROLOGY DIALYSIS TRANSPLANTATION, Bd. 18, Nr. 4, April 2003 (2003-04), Seiten 759-768, XP002515939 ISSN: 0931-0509
- William G. Goodman: "VITAMIN D IN HEALTH AND DISEASE: The Evolution of Assays for Parathyroid Hormone", Seminars in Dialysis, vol. 18, no. 4, 1 July 2005 (2005-07-01), pages 296-301, XP055213546, ISSN: 0894-0959, DOI: 10.1111/j.1525-139X.2005.18405.x
- HOCHER BERTHOLD ET AL: "Measuring Parathyroid Hormone (PTH) in Patients with Oxidative Stress - Do We Need a Fourth Generation Parathyroid Hormone Assay?", PLOS ONE, vol. 7, no. 7, July 2012 (2012-07), ISSN: 1932-6203
- L. COUCHMAN ET AL: "Mass spectrometric immunoassay for intact parathyroid hormone: correlation with immunoassay and application to clinical samples", ANNALS OF CLINICAL BIOCHEMISTRY, vol. 50, no. suppl 1, T18, 2013, page 27,
- COUCHMAN LEWIS ET AL: "LC-MS candidate reference methods for the harmonisation of parathyroid hormone (PTH) measurement: a review of recent developments and future considerations.", CLINICAL CHEMISTRY AND LABORATORY MEDICINE : CCLM / FESCC 1 SEP 2014, vol. 52, no. 9, 1 September 2014 (2014-09-01), pages 1251-1263, ISSN: 1434-6621
- P Ureña Torres: "The need for reliable serum parathyroid hormone measurements", Kidney International, vol. 70, no. 2, 1 July 2006 (2006-07-01), pages 240-243, XP055213543, ISSN: 0085-2538, DOI: 10.1038/sj.ki.5001658
- LOGUE FC: J. IMMUN. METH., vol. 39, 1991, page 159,
- LEPAGE R. ET AL.: CLIN. CHEM., vol. 44, 1998, pages 805-809,
- JOHN MR ET AL.: J. CLIN. ENDOCRINOL METAB., vol. 84, 1999, pages 4287-4290,
- GAO P ET AL.: POSTER M455, ASBMR 22ND ANNUAL MEETING, 2000,
- SLATOPOLSKY EDUARDO ET AL: "A novel mechanism for skeletal resistance in uremia", JOURNAL OF THE AMERICAN SOCIETY OF NEPHROLOGY, vol. 10, no. PROGRAM AND ABSTR. ISSUE, September 1999 (1999-09), page 625A, & 32ND ANNUAL MEETING OF THE AMERICAN SOCIETY OF NEPHROLOGY; MIAMI BEACH, FLORIDA, USA; NOVEMBER 1-8, 1999 ISSN: 1046-6673

## Beschreibung

Die Erfindung betrifft einen immunologischen Testkit zur Bestimmung der in einer Probe enthaltenen wirksamen Konzentration an Parathormon. Die Erfindung betrifft insbesondere ein Testbesteck zur Bestimmung des Gehalts an aktivem: Parathormon und dessen biologisch aktiven Fragmenten zur Diagnose, Ursachenfindung und Behandlung von Calciumstoffwechselstörungen, Osteopathien und Hyper- oder Hypoparathyreoidismen.

Das Parathormon (PTH) wird in der Nebenschilddrüse (Glandulae parathyroideae) gebildet und in den Blutkreislauf sezemiert. Es besteht in der intakten Form aus einer einzigen Polypeptidkette mit 84 Aminosäuren und besitzt ein Molekulargewicht von ca. 9500 Dalton (siehe SWISS-PROT: P01270, PTHY-HUMAN). Es bewirkt zusammen mit Vitamin D und Calcitonin die Mobilisierung von Calcium und Phosphat aus dem Knochenskelett und steigert die Aufnahme von Calcium im Darm sowie die Phosphatausscheidung über die Nieren. Die wirksame PTH-Aktivität in Plasma oder Serum ist daher ein wichtiger diagnostischer Parameter und erforderlich zur Klärung von 1) Vorliegen und Ausmaß eines Hyper- oder Hypoparathyreoidismus, 2) der Quantifizierung der Osteoblastenaktivität, 3) der Quantifizierung der Osteoklastenaktivität, 4) der Absicherung der Versorgung mit Vitamin-D und aktiven Vitamin-D-Metaboliten, 5) der Abschätzung einer Aluminiumbeladung, 6) der Abschätzung eines eventuellen Östrogenmangels bei postmenopausalen Dialysepatientinnen, 6) der notwendigen Steroid- oder Cydosporingabe nach Nierentransplantationen, 7) des Behandlungsbedarfs und der Vorbeugung von Knochenmarksveränderungen, urämischen Zuständen und chronischem Nierenversagen.

Die Bestimmung von PTH erfolgt im Stand der Technik durch den Nachweis von zwei entfernt liegenden Epitopen auf der PTH-Polypeptidkette. Mit der Einführung spezifischer hochsensitiver Immunoassays zur Bestimmung der Menge intakter PTH-Ketten in Humanserum schienen somit alle diagnostischen Fragen im Bereich der Calciumhomöostase, des Knochenumbaus und der übergeordneten Regelorgane wie der Nebenschilddrüse beantwortet,

Die so ermittelten Werte korrespondieren aber oft nicht zur tatsächlich vorhandenen PTH-Aktivität in der Plasma- oder Serumprobe, denn sie liefern nämlich oft PTH-Gehalte, die nicht vereinbar sind mit den Krankheitsbildern der Patienten. Bei Urämikern mit normalem Knochenumbau liegen die Serumspiegel von intaktern PTH zumeist um den Faktor 2,5 höher als bei Nierengesunden (pathologische Grenze bei Nierengesunden: 65 Mikrogramm PTH/L; bei Urämikern: 165 Mikrogramm PTH/L Serum). Zudem zeigen Urämiker mit vergleichbar hohen PTH - Werten oft deutliche Unterschiede im Knochenumbau (Slatopolsky E et al. (2000), Kidney Int. 58, 753-761). So haben diese Patienten im Serum oft acht- bis zehnfach erhöhte PTH-Konzentrationen, aber niedrignormale Werte für knochenspezifische alkalischer Phosphatase (Ostase). Sie sind also frei sind von Symptomen einer überhohen PTH-Aktivität. Als mögliche Ursachen werden diskutiert entweder eine systematische Fehlbestimmung oder eine PTH-Resistenz der Osteoblasten, beispielsweise durch genetisch verminderte Expression von PTH-Rezeptor, Weiter wird die Bestimmung des wirksamen PTH-Gehalts in Serum oder Plasma dadurch erschwert, dass das Hormon in der Zirkulation bzw. der Leber innerhalb von Minuten in aktive und inaktive Fragmente zerlegt wird. Einige dieser Fragmente haben eine Aktivität vergleichbar intaktem PTH (siehe EP-A 0 349 545; Schmidt-Gayk et al. (1999) Osteologie forum, 5, 48-58), andere wiederum wirken antagonistisch. So weiß man, dass die Fragmente hPTH(3-34) und hPTH(7-34) die Wirkungen des PTH inhibieren können (Suva et al, (1987) Science, 237, 893ff; EP 0 451 867),

Ferner werden hinter den Fehlbestimmungen "non-(1-84)" Large-PTH-Fragmente vermutet (LePage R, et al. (1998), Clin. Chem., 44, 805-809). Die Bezeichnung "Large-PTH-Fragment" steht allgemein für Fragmente, die mindestens die Polypeptidkette mit den Aminosäuren 39-84 und Epitope der N-terminalen Fragmente hPTH(1-33), hPTH(1-34), hPTH(1-37) oder hPTH(1-38) enthalten, denen aber die N-terminalen Aminosäuren 1 bis 3 fehlen. WO 96/10041 lehrt, dass nach Verlust der N-terminalen Aminosäuren Serin und Valin die PTH-Fragmente nicht mehr aktiv sind. Die Charakterisierung verschiedener immunologischer Tests mit synthetischen Fragmenten des Typs hPTH(7-84) ergab, dass diese in der Regel das inaktive Large-PTH(7-84)-Fragment mitbestimmen (John MR et al. (1999), J. Clin. Endocrinol, Metab.,. 84, 4287-4290; Gao P et al. 2000, Poster M455, ASBMR 22nd Annual Meeting; Roth HJ et al. (2000), Poster P1288; 11th International Congress of Endocrinology, Sydney). Die mitbestimmten inaktiven Large-PTH-Fragmente werden daher verantwortlich gemacht für die Diskrepanz zwischen gemessener PTH-Konzentration und klinischem Befund. Es wird vermutet, dass intakte PTH-Moleküle mit den Large-PTH-Fragmenten um die Bindungsstellen am PTH-Rezeptor konkurrieren und dass je nach Konzentration von CAP (Cydase activating PTH - biologisch aktiv) bzw. CIP (Cyclase Inhibiting PTH - biologisch inaktiv, Rezeptor-blockierend) es zu unterschiedlichen Zellaktivierungen kommt.

Es ist Aufgabe der Erfindung, einen Testkit zur quantitativen Bestimmung des wirksamen PTH-Gehalts einer Probe zur Verfügung zu stellen:
Diese Aufgabe wird gelöst durch den Testkit nach Anspruch 1. Weitere vorteilhafte Verwendungen des Testkits sind in den Unteransprüchen beschrieben.

Das Verfahren zur quantitativen Bestimmung des PTH-Gehalts in einer Probe ist dadurch gekennzeichnet, dass intakte PTH-Polypeptidketten und N-terminal-intakte Fragmente hiervon mit der PTH-Rezeptor-Bindungsstruktur (15 bis 22), die an einer oder mehreren Stellen nächst der PTH-Rezeptor-Bindungsstruktur (15 bis 22) oxidiert sind, von der Gehaltsbestimmung ausgenommen werden. Mit anderen Worten, ausgenommen werden von der Bestimmung der PTH-Aktivität bzw. des wirksamen PTH-Gehalts aller PTH-Polypeptidketten und Fragmente hiervon, die an Methionin-8 oder -18 oder an beiden Positionen oxidiert sind. Dies erfolgt durch den Einsatz von Antikörpern, die an ein PTH-Epitop binden, das von mindestens einer oxidierten Methionin-Gruppe an Position 8 und/oder 18 gebildet, wird, oder von Antikörpern, die spezifisch PTH-Polypeptidketten oder Fragmente hiervon binden, die an Methionin 8 und/oder 18 oxidiert sind, oder von Antikörpern, die spezifisch Konformationsepitope des oxidierten bzw. des "nativen" nicht-oxidierten Parathormons unterscheiden können. Es wird also sichergestellt, dass von der Bestimmung ausgenommen sind: inaktive Large-PTH-Fragmente des Typs hPTH(7-34) oder hPTH(3-84),, oxidiertes Intakt-hPTH(1-84) und oxidierte N-terminal-intakte oxidierte hPTH-Fragmente des Typs ox-hPTH(1-34) oder ox-hPTH(1-37), welche die Rezeptorbindungsstruktur PTH(15-22) enthalten.

Beschreibungsgemäß sind die Antikörper über eine Aufreinigung oder Selektion an Parathormon erhältlich, das an Position 8 und/oder 18 oxidiert ist. Die Antikörper können durch Immunisierung mit synthetischen und/oder speziell oxidierten PTH-Fragmenten gewonnen werden (Logue FC et al. (1991), Ann. Clin. Biochem., 28 160-166; Journal of Immunological Methods, 39, 159). Bspw. kann synthetisches PTH(1-34) durch Zusatz von H₂O₂ an den Methionin 8 und 18 oxidiert und für eine Immunisierung eingesetzt werden. Besonders bevorzugt sind monoklonale Antikörper gegen Methionin-8, welches anscheinend zuerst oxidiert wird.

In einer Ausführungsform des Verfahrens werden das Parathormon und dessen potentiell aktiven Fragmente chemisch nach-oxidiert und/oder nach-reduziert und der Gehalt an intaktem Parathormon und dessen aktiven Fragmenten über eine Doppelbestimmung (vorher und nachher) bestimmt. In einer anderen Ausführungsform wird getrennt zusätzlich der Gehalt an oxidiertem intakten Parathormon und Fragmenten hiervon in der Probe bestimmt und über die gleichzeitige Bestimmung von Gesamt-PTH und der Gehalt an nicht-oxidiertem aktiven PTH und dessen biologisch aktiven. Fragmenten berechnet.

In einer besonders bevorzugten Ausführungsform des Tests werden Antikörper gegen an Position 8 und/oder 18 oxidiertes Parathormon zur Maskierung des oxidierten Parathormons zugesetzt, so dass ausschließlich nicht-oxidiertes bzw. biologisch aktives Parathormon und dessen Fragmente vom Primär- und Sekundär-Antikörper gebunden werden. Der erfindungsgemäße Test umfasst dann folgende Schritte: Umsetzen der Probe mit einem Antikörper, der ein Epitop erkennt, an denn an Position 8 und/oder 18 oxidiertes Parathormon beteiligt ist; Umsetzen der Probe mit einem Antikörper, der ein Epitop erkennt, das von den N-terminalen Aminosäuren 1 bis 3 des Parathormons gebildet wird; Umsetzen der Probe mit einem Antikörper, der ein Epitop erkennt im Bereich der Rezeptorbindungsstruktur (Aminosäuren 15 bis 22) des humanen Parathormons und der nicht binden kann, wenn ein Antikörper bereits im Bereich der oxidierte Methionine 8 und/oder 18 gebunden hat, und Bestimmen der Zahl der Moleküle, die von beiden Antikörpern erkannt werden. Bevorzugt erfolgt hierbei die Bindung der beiden Antikörper in Gegenwart von 0,05 bis 0,1 Gew.% eines leichten Detergens wie Tween™ 20 oder Triton™ X-100 erfolgt. Erfindungsgemäß wird also in einer besonders bevorzugten Ausführungsform die Probe zunächst mit Antikörpern gegen oxidiertes Parathormon versetzt, so dass Antikörper, die im Bereich der Rezeptorbindungsstelle des Parathormons binden, nicht an die oxidierte PTH-Polypeptidkette binden können. Der Fachmann erkennt, dass die Antikörper zur Maskierung von oxidiertem Parathormon und Fragmenten hiervon dann besser von einem anderen Typ sein sollten als die Antikörper zum Aufbau bzw. Nachweis des Sandwich-Komplexes. Die als Fänger und Tracer eingesetzten Antikörper können, wie der Fachmann erkennt, auch funktionell vertauscht werden.

In einer weiteren Ausführungsform trägt mindestens einer der Antikörper eine Markierung, ausgewählt aus einer fluoreszierender oder chemilumineszierenden Gruppe oder einem Enzym zur Katalyse einer Nachweisreaktion. Bevorzugt ist einer der Antikörper ein mit einem Ruthenium-Komplex markierter monoklonaler Antikörper, der ein Epitop im Bereich der Aminosäuren 15 bis 22 oder 26 bis 32 erkennt. Einer der Antikörper kann auch biotinyliert sein und über Streptavidin an eine feste Phase gebunden werden. In dem Verfahren können zudem Antikörper eingesetzt werden, welche an die PTH-Polypeptidkette mit Aminosäuren 4 bis 14 oder 15 bis 35v39 binden. Ein Antikörper, der an ein Epitop im Bereich der Aminosäuren 15 bis 22 erkennt, ist besonders bevorzugt, da einerseits dieses Epitop im Bereich der Rezeptorbindungsstruktur liegt, anderseits ein solcher Antikörper wegen dem Methionin-18 zwischen oxidiertem und nativem hPTH unterscheiden kann.

Das erfindungsgemäße Testsystem zur quantitativen Bestimmung von Parathormon und Fragmenten hiervon in einer Probe umfasst in einer Ausführungsform an einer Phase gebundene Antikörper (Fänger-Antikörper), die in oder nächst der PTH-Rezeptor-Bindungsstruktur des Parathormons binden und - zur Maskierung - freie (ungebundene) Antikörper, die Epitope des Parathormons mit oxidiertem Methionin 8 und/oder 18 binden. Die Antikörper können durch Affinitätschromatographie aufgereinigt werden, bspw. über ihre Bindung an Methionin 8 und/oder 18 des oxidierten Parathormons. Als Tracer-Antikörper werden Antikörper gegen die N-terminale Aminosäuren 1 bis 3 des Parathormons (in der reduzierter Struktur) verwendet, Fänger- und Tracer-Antikörper können auch vertauscht werden.

Die Beschreibung betrifft weiterhin die Diagnose von Oxidationsstress bei Dialysepatienten, wobei ein vitro Test zur quantitativen Bestimmung von Parathormon und dessen aktiven Fragmenten gemäß der Erfindung eingesetzt wird.

Wenngleich man aus den Untersuchungen an synthetischen PTH-Fragmenten wusste, dass oxidiertes Parathormon bzw. oxidierte PTH-Fragmente biologisch inaktiv sind, so gab es keine Vorstellung, dass diese Oxidation physiologisch relevante Ausmaße annehmen kann und die Bestimmung der wahren physiologischen PTH-Aktivität in einer Probe, verhindert. So werden in einigen herkömmlichen Testverfahren Reduktionsmittel zur Stabilisierung der Antikörper in den Probenpuffer zugesetzt, in anderen eine nachträgliche partielle Oxidation der PTH-Moleküle in Kauf genommen.

Die Beschreibung betrifft zudem in einer Ausführungsform ein Verfahren zur Diagnose und Bestimmung des Ausmaß eines Hypo- oder Hyperparathyreodismus, der Ursachenfindung von Calciumstoffwechselstörungen, Osteopathien, Nierenversagen und Zuständen, die von einer gestörten Homeostase des Calcium- und Phosphatgehalts des Bluts herrühren. Weiterhin wird erfindungsgemäß die direkte Produktion N-terminaler PTH-Fragmente, sofern gegeben, miterfasst. Das heißt, die erfindungsgemäße PTH-Diagnostik erfasst neben dem intaktem aktiven Parathormon auch direkt produzierte, biologisch aktive PTH-Fragmente.

Sauerstoff ist für das aerobe Leben essentiell, zugleich aber das wichtigste Vorläufermolekül von freien reaktiven Sauerstoffspezies (ROS), die. durch ihre Reaktion mit Proteinen, Lipiden und Nukleinsäuren die Zellen schädigen und durch die Oxidation reaktiver Aminosäuren Proteine in ihrer Struktur verändern können. Eine Vielzahl Publikationen beschäftigt sich zwar mit den Auswirkungen des Oxidationsstress und dem Einfluss der "Advanced glycation end products" auf die Pathogenese der Komplikationen bei chronisch niereninsuffizienten Patienten (Martin-Mateo MC et al, (1999), Ren. Fail., 21, 155-167; Hasselwander O et al. (1998), Free Radio. Res., 29, 1-11; Zoccali C et al. (2000), Nephrol. Dial. Transplant., 15, Suppi.2; Canaud B et al. (1999), Biood Purif., 17, 99-106). Dialysepatienten sind im Vergleich zu Normalpersonen weit stärker vom oxidativen Stress belastet. Verschiedene Arbeitsgruppen haben oxidiertes Parathormon und dessen biologische Aktivität untersucht (Alexiewicz LM et al. (1990), J, Am. Soc. Nephral., 1, 236-244; Zull JE et al.(1990), J. Biol. Chem., 265, 5671-5676; Pitts TO et al. (1988), Miner. Electrolyte Metab., 15, 267-275; Horiuchi N. (1988), J. Bone Miner. Res., 3, 353-358; Freilager AL et al. (1986) Arch. Biochem. Biophys., 244, 641-649; Galceran T et al. (1984) Endocrinology 115, 2375-2378; Frelinger AL et al. (1984), J. Biol, Chem., 259, 5507-5513; O'Riordan JLH et al. (1974), J. Endocr., 63. 117-124; Logue FC et al. (1991),. Ann. Clin. Biochem. 28, 160-166; Logue FC (1991b), J. Immun. Meth., 39, 159). Der Oxidationsstress bei Dialysepatienten und dessen Folgen auf die PTH-Diagnostik wurde aber bislang nicht untersucht und erkannt.

Es wurde nun gefunden, dass die herkömmliche Bestimmungsverfahren je nach Testbesteck und Antikörpern sowohl bioaktives (natives bzw. reduziertes) intaktes PTH und ggf. dessen Fragmente als auch oxidiertes PTH und ggf. dessen Fragmente erfassen und somit, den wirksamen Gehalt an Parathormon falsch bestimmen.

Ferner wurde gefunden, dass die postulierten Large-PTH-Fragmente, sofern es sie überhaupt gibt, keine biologische Bedeutung besitzen, sondern dass die Diskrepanz zwischen gemessenem PTH-Gehalt und medizinischem Befund zumindest partiell vom Oxidationsstress herrührt, dem Dialysepatienten unterliegen. Der wirksame PTH-Gehalt. in einer Plasma- oder Serumprobe ist nämlich abhängig vom Oxidationsstress und der Oxidationsstress zugleich eine Ursache von pathologischen Zuständen.

Die Beschreibung betrifft ferner ein Verfahren, bei dem ein erster Fänger-Antikörper an eine feste Phase gebunden ist. Der zweite Tracer-Antikörper kann eine Markierung tragen, z.B. eine Fluoreszenz- oder Chemilumineszenzgruppe, oder konjugiert sein, mit einem Enzym wie alkalische Phosphatase oder Peroxidase. Das Verfahren erfolgt erfindungsgemäß in einem an sich bekannten Immunoassay, vorzugsweise in einem ELISA, ECLIA, IEMA, IRMA, ILMA oder LIA. Die Bindung der beiden Antikörper an das PTH erfolgt bevorzugt in Gegenwart von 0,05 bis 0,1 Gew.% eines milden Detergens wie Tween™-20 oder Triton™X-100, so dass eine Faltung des N-terminalen Epitops PTH(1-3) zur PTH-Rezeptor-Bindungsstruktur verhindert ist bzw. um das N-terminale Epitop PTH(1-3) für die Bindung des Antikörpers zugänglich zu halten. Da für die biologische Aktivität und die Faltung des PTH sowohl die Rezeptorbindungsstruktur als auch das aminoterminale Epitop PTH(1-3) nötig sind, wechselwirken diese beiden Strukturen vermutlich miteinander. Durch die Gegenwart eines milden Detergens kann in vorgenannten Ausführungsformen mit Antikörpern gegen hPTH(1 -3) die Empfindlichkeit und Genauigkeit des Assays deutlich verbessert werden.

In einer Ausführungsform des Verfahrens wird ein bekanntes Aliquot der Probe zudem mit Antikörpern umgesetzt, die an die PTH-Sequenzen zwischen Aminosäure 4 bis 14 und/oder 15 bis 37 binden. Die Zahl der Moleküle in einer Probe, die Antikörper gegen das Epitop PTH(1-3) und die Rezeptorbindungsstruktur des PTH binden, und die Zahl der Moleküle, die Antikörper gegen die PTH-Bereiche 4 bis 14 und 15 bis 37 binden, werden dann zueinander in Beziehung gebracht und die biologisch wirksame PTH-Aktivität ermittelt.

Die Erfindung betrifft weiterhin ein Diagnosesystem zur Bestimmung der PTH-Aktivität in einer Probe, das gekennzeichnet ist durch Antikörper, die spezifisch das N-terminale Epitop mit den Aminosäuren 1 bis 3 erkennen und Antikörper, die an den Bereich der PTH-RezeptorBindungsstruktur binden, in einer anderen Ausführungsform weist das Diagnosesystem zudem Antikörper auf, die an den Bereich zwischen Aminosäure 4 und 14 binden. In einer dritten Ausführungsform beinhaltet das System Antikörper, die an den Abschnitt zwischen Aminosäure 24 und 37 des Parathormons binden oder an den Mitten- (53 bis 68) oder den C-terminalen (53 bis 84) Abschnitt der PTH-Polypeptidkette.

Das beschreibungsgemäße Verfahren berücksichtigt im Gegensatz zu früheren Immunoassays die PTH-Aktivität auch der nicht-intakten PTH-Fragmente und dass scheinbar intaktes PTH biologisch inaktiv ist, wenn die letzte oder die letzten beiden aminoterminalen Aminosäuren des Peptidhormons fehlen oder wenn ein oder beide Methionine an Position 8 und 18 des N-terminalen Fragments oxidiert sind. PTH-Fragmente, die weder eine Rezeptor-Bindungsstruktur mit den Aminosäuren 15 bis 22 noch ein intaktes N-terminales Epitop PTH(1-3) aufweisen, besitzen keine agonistische oder antagonistische Aktivität.

Herkömmliche immunologische Tests zur quantitativen Bestimmung von intaktem Parathormon oder hPTH(1-37) geben falsche Aktivitätswerte, wenn nur aus dem Vorhandensein von zwei Epitopen auf eine physiologische Aktivität geschlossen wird. Herkömmliche Verfahren beachten nicht, dass die richtige Faltung bzw. die Bindung an den PTH-Rezeptor von einem intaktem Aminoterminus mit den Aminosäuren Serin und Valin abhängig ist und dass die Methionin-Gruppen an Position 8 und 18 nahe der PTH-Rezeptor-Bindungsstruktur nicht oxidiert sein dürfen. Die Erfindung beruht insbesondere auf der Entdeckung, dass die Oxidation des PTHs an den vorgenannten Positionen, insbesondere bei Dialysepatienten, erheblich sein kann und diagnostisch bedeutsam ist. Die in herkömmlichen Tests als aktives PTH bestimmten Fragmente hPTH(7-84), hPTH{3-84) oder hPTH(4-37) - sofern vorhanden - besitzen keine bzw. keine messbare antagonistische Aktivität. Testsysteme auf der Basis von Antikörpern gegen den Mittenabschnitt (53-68) oder den C-terminalen (53-84) Abschnitt übersehen, dass manche Patienten direkt biologisch aktive PTH-Fragmente des Typs hPTH(1-37), hPTH(1-32-36) oder hPTH(1-38) produzieren.

Auch immunologische Verfahren auf der Grundlage von Antikörpern gegen hPTH{1-3) bzw. hPTH(1-6) bzw. gegen Peptide aus dem hPTH(1-37)-Fragment spiegeln falsche Aktivitäten vor, denn es werden nicht nur die biologisch aktiven (reduzierten) Struktureinheiten bestimmt. Mit polyklonalen Antikörpern oder Antiseren gegen das N-terminale Peptid mit den Aminosäuren 1 bis 5 oder 6 werden teilweise sogar antagonistisch wirkende Fragmente als aktive PTH-Fragmente erkannt.

Das Verfahren stellt somit dem Arzt einen PTH-Aktivitätswert zur Verfügung, der die physiologische Wirkungen des nativen (reduzierten) Parathormons und seiner aktiven Fragmente beschreibt. Es erkennt auch Patienten mit Hyperparathyreodismus, die normale oder verminderte Gehalte an intaktem hPTH(1-64) im Serum besitzen und direkt PTH-Fragmente wie hPTH (1-37) und hPTH (1-38) in den Blutstrom sezemieren.

Das beschreibungsgemäße Verfahren zur Bestimmung der Aktivität von PTH und seinen Fragmenten in einer Probe kann verwirklicht werden als EIA, ELISA, RIA, IRMA, LIA oder ICLIA, ILMA, FIA oder IFMA, als ein manuelles Testsystem oder bevorzugt in einer auf Automaten angepasste Version in Flüssigphasen- oder Festphasentechnik.

Weitere Vorteile, Merkmale und Ausführungsformen der Erfindung ergeben sich aus den nachstehenden Beispielen und den anliegenden Zeichnungen. Es zeigt:
- Fig. 1: ein Diagramm mit Resultaten (Testsystem: Elecsys™ 2010 der Roche Diagnostics GmbH, Mannheim) der Spiking-Versuche am PTH-Standard;
- Fig. 2: ein Diagramm mit Resultaten (Elecsys™ 2010 der Roche Diagnostics) der Spiking-Versuche an Poolplasmen;
- Fig. 3: ein Diagramm mit Resultaten (Testsystem: PTH-IRMA von Nichols Institute, U.S.A.) der Spiking-Versuche am PTH-Standard;
- Fig. 4: ein Diagramm mit Resultaten (PTH-IRMA von Nichols Institute) der Spiking-Versuche an Poolplasmen;
- Fig. 5: ein Diagramm mit Messwerten (Roche Elecsys™ 2010) der Plasmaproben nach Oxidation mit H₂O₂;
- Fig. 6: ein Diagramm mit prozentualen Messwerten (Roche Elecsys™ 2010) der Plasmaproben nach Oxidation mit H₂O₂;
- Fig. 7: ein Diagramm mit Messwerten (PTH-IRMA von Nichols Institute) der Plasmaproben *nach* Oxidation mit H₂O₂;
- Fig. 8: ein Diagramm mit prozentualen Messwerten (PTH-IRMA von Nichols Institute) der Plasmaproben nach Oxidation mit H₂O₂;
- Fig. 9: ein Diagramm mit Messwerten (PTH-IRMA von. Nichols Institute) nach Oxidation und Rückreduktion der Proben;
- Fig. 10: ein Diagramm mit prozentualen Messwerten. (PTH-IRMA von Nichols Institute) nach Oxidation und Rückreduktion der Proben;
- Fig. 11: ein Diagramm mit prozentualen Messwerten (PTH-IRMA von Nichols Institute) nach Oxidation und direkter Reduktion der Proben;
- Fig. 12: ein Diagramm mit prozentualen Messwerten (PTH-IRMA von Nichols Institute) von einer Plasmaprobe mit und ohne Oxidation und nach Zusatz von monoklonalen Antikörpern gegen oxidiertes Parathormon;
- Fig. 13: ein Diagramm mit prozentualen Messwerten von einer Plasmaproben (Roche Elecsys™ mit und ohne Oxidation und nach Zusatz von monoklonalen Antikörpern gegen oxidiertes Parathormon.

### BEISPIELE

### Beispiel 1 Biologische Wirkung der Large-PTH-Fragmente

Es wurde untersucht, ob die im Stand der Technik postulierten Large-PTH-Fragmente in Humanserum Vorkommen und eine antagonistische Wirkung auf den PTH-Rezeptor besitzen. Die verwendeten Immunoassay beruhten auf Antikörpern, die nicht mit synthetischem hPTH(7-84) (Bachem AG) kreuzreagieren, so dass die Messwerte die wahre Konzentration von intaktem, aktiven hPTH in Humanserum angeben sollten. Diese Werte wurden mit Parametern für den Knochenumbau verglichen. Die Produktion der Large-PTH-Fragmente wird gemäß Stand der Technik über einen Rückkopplungsmechanismus geregelt und bei erhöhtem oder vermindertem Bedarf führt die Stimulation bzw. Suppression der Nebenschilddrüse zu einer adäquaten Ausschüttung, Derzeit sind zwei Testsysteme zur quantitativen Bestimmung von PTH bekannt, die keine klinisch relevante Kreuzreaktivität, mit dem synthetischen Large-PTH-Fragment hPTH(7-84) besitzen. Dies sind der Elecsys™ 2010-PTH-Immunoassay von Roche Diagnostics GmbH, Mannheim und der CAP-PTH-IRMA von der Firma Scantibodies. Der Elektrochemilumineszenz-Immunoassay (Elecsys™ von Roche Diagnostics GmbH, Mannheim, beruht auf einem monoklonalen Fänger-Antikörper gegen ein N-terminales PTH-Epitop entsprechend den Aminosäuren 26-32 und einem Ruthenium-Komplex markierten Tracer-Antikörper gegen ein C-terminales hPTH-Epitop entsprechend den Aminosäuren 55-64. Das verwendete synthetische Large-hPTH(7-84)-Fragment (Bachem AG) wurde nicht vom Fänger-Antikörper des Elecsys™ 2010« PTH-Immunassays erkannt, so dass er eine tertiäre Faltungsstruktur erkennen muss bzw, ein sogenannter Konformationsantikörper ist. Vermutlich war aber das eingesetzte Large-hPTH(7-84)-Fragment (Gao P et al. 2000, Poster M455, ASBMR 22nd Annual Meeting; Roth HJ et al. (2000), Poster P1288; 11th International Congress of Endocrinology, Sydney), wie die jetzigen Ergebnisse nahelegen, bei der Synthese oder Lagerung oxidiert worden. Der Fänger-Antikörper des Elecsys™ 2010-PTH-Immunassays bindet vermutlich nur oxidiertes hPTH(7-84) nicht, reduziertes PTH(7-84) aber schon. Der CAP-PTH-IRMA der Firma Scantibodies verwendet polyklonale Antikörper gegen das N-terminale hPTH(1-6), die das Large-PTH(7-84)-Fragmente nicht binden können.

Neben der Bestimmung von intaktem PTH mit verschiedenen Immunoassays (Nichols-IRMA, Roche Elecsys™ 2010, DUO-PTH Scantibodies, PTH-Immundiagnostik) wurden auch die Knochenmarker BAP (Ostase) als Formationsmarker und TRAPSb als Resorptionsmarker bestimmt. Die Ergebnisse sind in der nachstehenden Tabelle I gezeigt.

**TABELLE 1**

| Korrelation von Knochen-An- und Abbaumarker mit PTH-Messwerten | | |
|---|---|---|
| Abhängige Variable Y | Unabhängige Variable X | Korrelationskoeffizient / n= |
| BAP fOstasef | PTH CAP Scan, | 0.484 n=96 |
| BAP fOstaset | PTH Elecsvs2Q10 | 0.474 n=96 |
| BAP fOstasef | PTH Nichols | 0.485 n=96 |
| TRAP5b | PTH CAP Scan | 0.511 n=95 |
| TRAPSb | PTH Elecsys2010 | 0.510 n=95 |
| TRAP5b | PTH Nichols | 0.524 n=95 |
| PTH CAP Scan | PTH Elecsvs2010 | 0.966 n=103 |
| PTH CAP Scan | PTH Nichols | n 97P n=103 I |

Die Korrelationen der Werte für die An- und Abbaumarker und den PTH-Gehalten im Serum zeigen keine signifikant höheren Korrelationen für die spezifischeren Tests ("keine Kreuzreaktivität zu Large-PTH-Fragmenten") im Vergleich zum IRMA der Firma Nichols Institute. Untereinander ist die Korrelation der einzelnen PTH-Assays, wie bereits in anderen Studien belegt, ausgezeichnet, Dies zeigt, dass keines der bekannten Testsysteme zufriedenstellende Resultate liefert.

### Beispiel 2 Oxidations- und Reduktionsversuche

Es wurde untersucht, ob und inwieweit der Elecsys™ 2010-PTH-Test und der Nicols-Intakt-PTH-Test natives bzw. oxidiertes PTH erkennen.

Der Nichols-Intakt-PTH-Assay (Nichols Institute, San Juan Capistrano, California, U.S.A.) ist ein doppelt bindender Immunoradiometrie-Assay (IRMA) mit zwei polyklonale Ziegen-Antikörpern. Der auf Kunststoff-Beads immobilisierte Fängerantikörper bindet nur im Mitten- oder C-terminalen Abschnitt des hPTHs (anti-hPTH(39-89). Der zweite ^{12S}iod-markierte Nachweisantikörper erkennt ein Epitop im N-terminalen Abschnitt des PTHs (anti-hPTH(1-34). Die Probe wurde zeitgleich mit den Antikörper-beschichteten Beads und dem ¹²⁵Iod-markierte Nachweisantikörper über Nacht (12 ± 2 h) bei Raumtemperatur inkubiert. Nach der Inkubation wurden die Beads gewaschen und die an der festen Phase anhaftende Radioaktivität in einem Gamma-Zähler (LKB-1277 Gammamaster von Perkin-Elmer) gemessen. Die Dosis-Messkurve (MultiCalc-Software) zwischen gebundener Radioaktivität und Konzentration in der Probe wurde streng nach Herstellerangaben über einen mitgemessenen Standard bestimmt. Die Interassay-Varianz im Normal- und erhöhten Bereich betrug weniger als 10% CV.

Der Roche Elecsys®-PTH-Assay ist ein Electrochemilumineszenz-Immunoassay (ECLIA) auf dem vollautomatischen Immunoanalyzer Elecsys® 2010. Das vom biotinylierten Fängerantikörper erkannte Epitop enthält die PTH-Aminosäuren 26 bis 32 und der monoklonale Ruthenium-Nachweisantikörper erkennt nach Angabe des Herstellers ein Epitop im Bereich der PTH-Aminosäuren 55 bis 64. Probe und Antikörper wurden zusammen 9 Minuten bei 37°C inkubiert, Streptavidin-beschichtete paramagnetische Beads zum Inkubationsgemisch zugegeben und schließlich weitere 9 Minuten inkubiert. Nach magnetischer Trennung der gebundenen von den freien Konjugaten auf einer Elektrodenoberfläche wurde die Lumineszenz bestimmt und die End-PTH-Konzentration über eine rekalibrierte Masterkurve ermittelt. Die Interassay-Varianz betrug im niederen und oberen Normalbereich weniger als 7% CV und im Hochkonzentrationsbereich 5% CV.

Für die Oxidations- und Reduktionsversuche wurden Standards bzw. Poolplasmen von Dialysepatienten mit hPTH(1-37)-Fragmenten unterschiedlicher Konzentrationen und Oxidationszustand versetzt. Alle Proben enthielt EDTA als Antikoagulanz. Die Messung der nativen, unveränderten Ausgangsprobe (Bezugsgröße) sowie die der versetzten Poolplasmen erfolgte zeitgleich. Die Bestimmungen erfolgten jeweils streng nach den Vorgaben der Hersteller.

### 1, Standardversuch

PTH-IRMA-Standard der Firma Nichols Institute mit einer nominalen Konzentration von 46,8 pmol/l.

### 2. Versuch mit Plasmapools

Es wurden zwei getrennte Proben aus EDTA-Plasmaproben von Dialysepatienten mit erhöhten Gehalten an intaktem PTH gepoolt.

| | | |
|---|---|---|
| Pool | PTH-Intakt Elecsys | PTH-intakt Nichols |
| Pool 1 | 76,7 pmol/l | 59,2 pmol/l |
| Pool 2 | 72,4 pmol/l | 54,5 pmol/l |

Die Standardprobe und die Plasmapools wurden jeweils versetzt mit drei verschiedenen Konzentrationen an hPTH(1-37)-Fragment in fünf verschiedenen Oxidations- und Zustandsformen und dann die wiedergefundenen PTH-Gehalte bestimmt.

Zwei zugesetzte PTH(1-37)-Fragmentlösungen enthielten natives hPTH(1-37) von verschiedenen Herstellern (1: IPF=Institut für Peptidforschung, Braunschweig, 2=Immundiagnostik AG, Bensheim). In der dritten Fragmentlösung des hPTH(1-37) war das Methionin an Position 8 oxidiert, in der vierten das Methionin an Position 18 und in der fünften beide Methionine an Position 8 und 18.

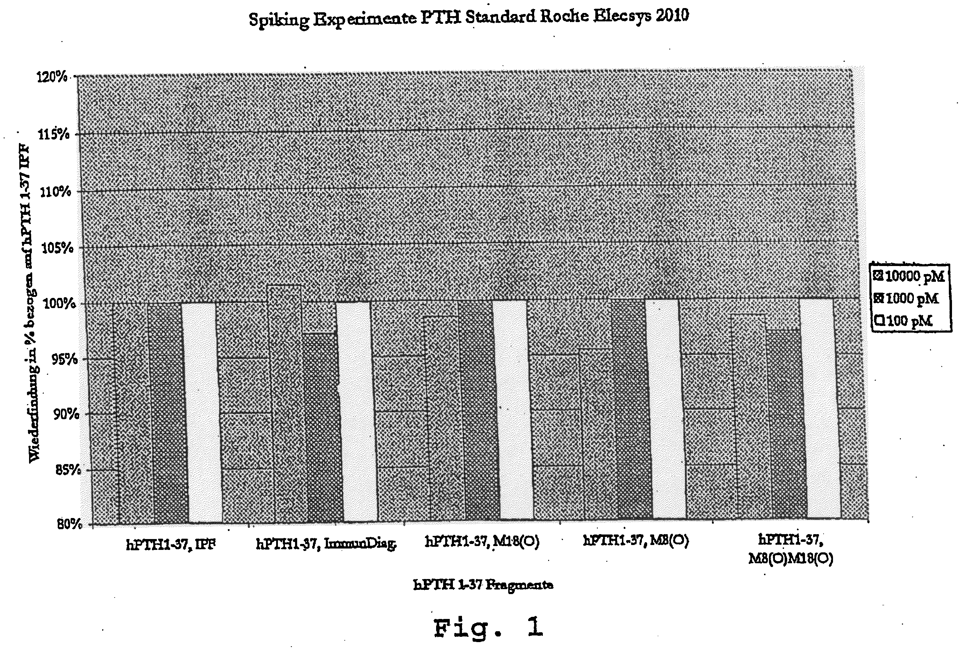

Es wurden von jeder Fragmentlösung drei Ausgangskonzentrationen (1000 nM, 100 nM und 10 nM) hergestellt. Die weitere Verdünnung der Fragmentlösungen 1:100 im Standard bzw. in den Poolplasmen ergab eine Endkonzentrationen 10 000 pMol, 1000 pMol und 100 pMol in der zu analysierender Probe. Es wurden jeweils drei Fragment-Konzentrationen eingesetzt, weil die Bindungskapazitäten der in den kommerziellen Tests verwendeten Fängerantikörper gegen das N-terminale PTH-Fragment nicht bekannt waren. Ziel des Versuchs war, die Fängerantikörper-Bindungsstellen mit nativem bzw. oxidiert hPTH(1-37) komplett zu belegen, so dass sie nicht mehr zur Ausbildung des Sandwich-Komplexes zur Verfügung stehen und das Messsignal unter die jeweilige Nachweisgrenze fällt, Der Vergleich der Messwerte von Proben mit zugesetztem nativen hPTH(1-37)-Fragment bzw. zugesetzten oxidierten Fragmenten gibt dann Auskunft über die Spezifität der Fänger-Antikörper. Die Ergebnisse sind in den in Figuren 1 bis 4 gezeigten Diagrammen zusammengefasst.

Zur Berechnung der Blockade des N-terminalen Antikörpers wurden die gemessenen Konzentrationen des Standards bzw. der Poolplasmen versetzt mit der nativen PTH-Präparation hPTH(1-37) von IPF *auf* 100% festgesetzt und alle weiteren Werte hierzu in Beziehung gesetzt.

### Diskussion der Ergebnisse mit dem Elecsys™ 2010-PTH-Test

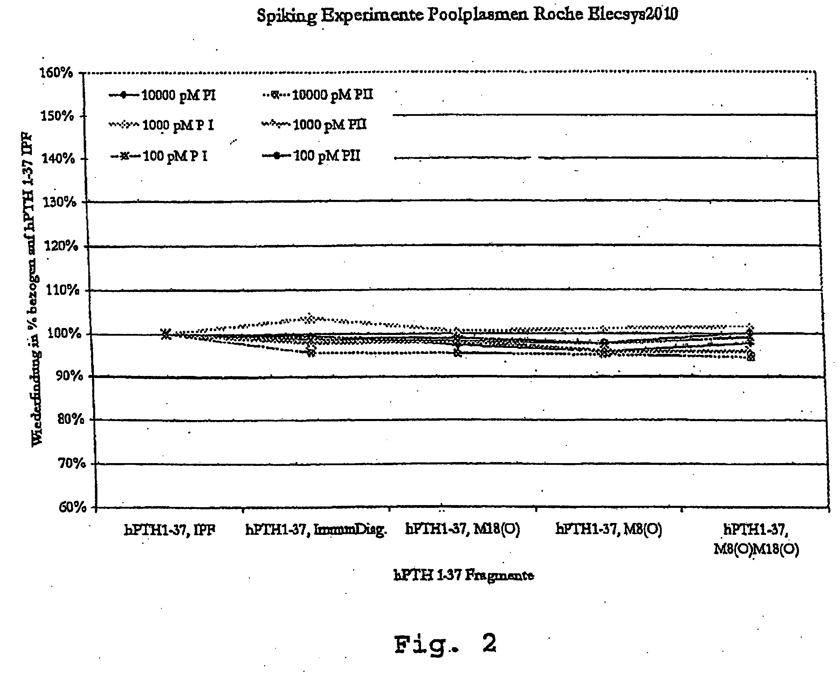

Siehe Diagramme der Figuren 1 und 2. Aufgrund der minimalen prozentualen Abweichungen der Messwerte aller, an unterschiedlichen Positionen oxidierten PTH-Fragmente, kann davon ausgegangen werden, dass der Roche Elecsys™ 201 Ö-Test das oxidierte PTH nur mit geringer Affinität erkennt und überwiegend mit nativem und somit biologisch aktivem Parathormon reagiert. Die fehlende Signalreduktion auch bei der sehr hohen Fragmentkonzentration von 10 000 pM spricht für eine extrem hohe Bindungskapazität des Fänger-Antikörpers. Die nativ Vermessenen Proben lagen in der Absolut-Konzentration maximal 10 % höher als die entsprechenden gespickten Proben. Nachteilig an dem Elecsys™ 2010-PTH-Test bleibt aber, dass direkt produzierte aktive N-terminale PTH-Fragmente nicht erfasst werden, da der Tracer-Antikörper im C-terminalen Abschnitt des PTH bindet, andererseits aber biologisch inaktive PTH(4-37)-Fragmente, denen das N-terminale Epitop mit den Aminosäuren 1 bis 3 fehlt, partiell miterfasst werden.

### Diskussion der Ergebnisse mit dem Nichols-IRMA-PTH-Test

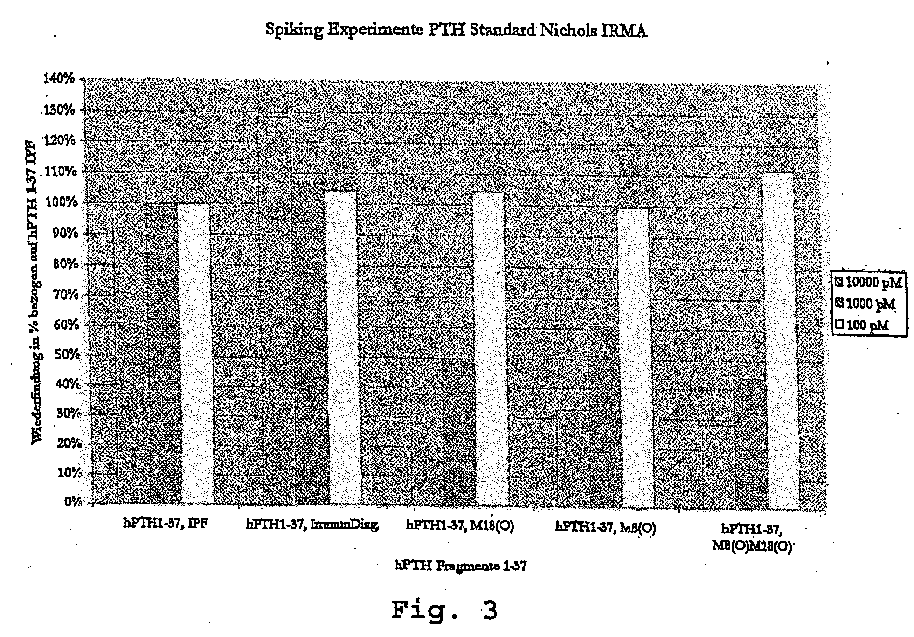

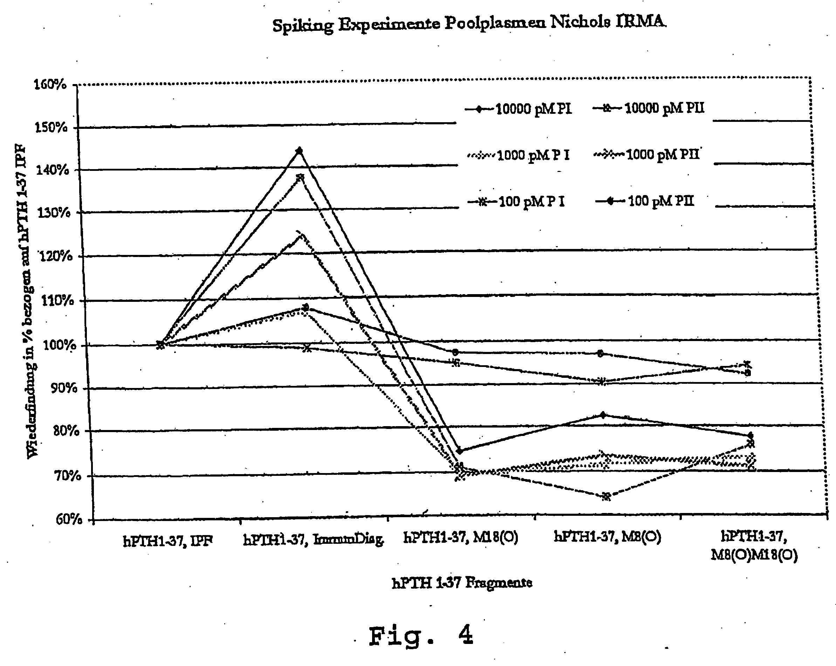

Siehe Diagramme *der* Figuren 3 und 4. Anders als beim Elecsys™ 2010-PTH-Test produzieren die Spiking-Versuche beim IRMA-Intakt-PTH-Test deutliche Unterschiede im Reaktionsverhalten nach Zugabe der verschiedenen Fragmente. Auch verhält sich der verwendete Standard different zu den Poolplasmen, was von der unterschiedliche Matrix (Proteingehalt etc,) bedingt sein dürfte. Die bei einer Konzentration von 1000 pM hPTH(1-37) sichtbare Signatreduktion auf 50% der nativ vermessenen Probe zeigt die niedrige Bindungskapazität der Fänger-Antikörper gegen das N-terminale PTH-Fragment. Die nicht-oxidierten hPTH(1-37)-Fragmente von IPF und Immundiagnostik verhalten sich unterschiedlich, wobei die Differenz bei den Poolplasmen besonders deutlich ist. Auch die *Messsignale nehmen* deutlich ab, sobald oxidierte hPTH(1-37)-Fragmente zugesetzt werden. Aufgrund der Ergebnisse kann davon ausgegangen werden, dass das im Testbesteck der Firma Nichols verwendete polyklonale Antikörpermaterial verschiedene Antikörperpopulationen enthält, die sowohl mit nativem als auch mit oxidiertem PTH reagieren. Das unterschiedliche Bindungsverhalten der verschiedenen oxidierten hPTH(1-37)-Fragmenten ist interessant. Es wird zu prüfen sein, ob die verschieden oxidierten Moleküle (an Position 8 oder 18 oder beide Positionen) unterschiedliche biologische Wirkungen besitzen.

Mit dem Experiment konnte nachgewiesen werden, dass mit dem polyklonalen Antikörpermaterial (Nichols-IRMA) im Gegensatz zum monoklonale n Antikörper (Elecsys *2010}* neben dem nativen PTH auch oxidierte inaktive PTH-Formen erfasst werden. Das oxidierte PTH besitzt an den Zielzellen für die Calciumhomeostase (Niere, Knochen) eingeschränkte bis keine biologische Aktivität.

### Beispiel 3 Oxidation und Reduktion von Plasmaproben urämischer Patienten

Dialysepatienten sind bei: der Dialyse einem erhöhten Oxidationsstress ausgesetzt. Es wurde daher untersucht, ob *in vivo* eine Oxidation des PTH erfolgt - mit. entsprechender Konsequenzen, für seine biologischen Aktivität, Es wurde weiterhin untersucht, ob und inwieweit eine direkte Oxidation bzw. eine Reduktion oder Rückreduktion der PTH-Molekülen in den Proben möglich ist.

Es wurden willkürlich 30 Plasmaproben von Dialysepatienten und ESRD-Patienten (ESRD=end-stage renal disease) ausgewählt und mit Wasserstoffperoxid (30%; Endkonzentration: 1,5 Mol/L) oxidiert bzw. mit Natriumborhydrid (NaBH₄, wässrige Lösung von 100nMol/L) reduziert. Alle Proben, behandelt wie unbehandelt, wurden zeitgleich mit dem Roche Elecsys™ 2010 und dem Nichols IRMA Test vermessen.

Die Oxidation erfolgt, mit 30%iger Wasserstoffperoxid-Lösung. Die WasserstoffperoxidKonzentration im Inkubationsansatz lag bei 17% der 30%igen Lösung. Alle Proben wurden für 45 Minuten bei 37°C inkubiert, dann auch -70°C abgekühlt, lyophilisiert und bis zur quantitativen PTH-Bestimmung bei -20°C gelagert.

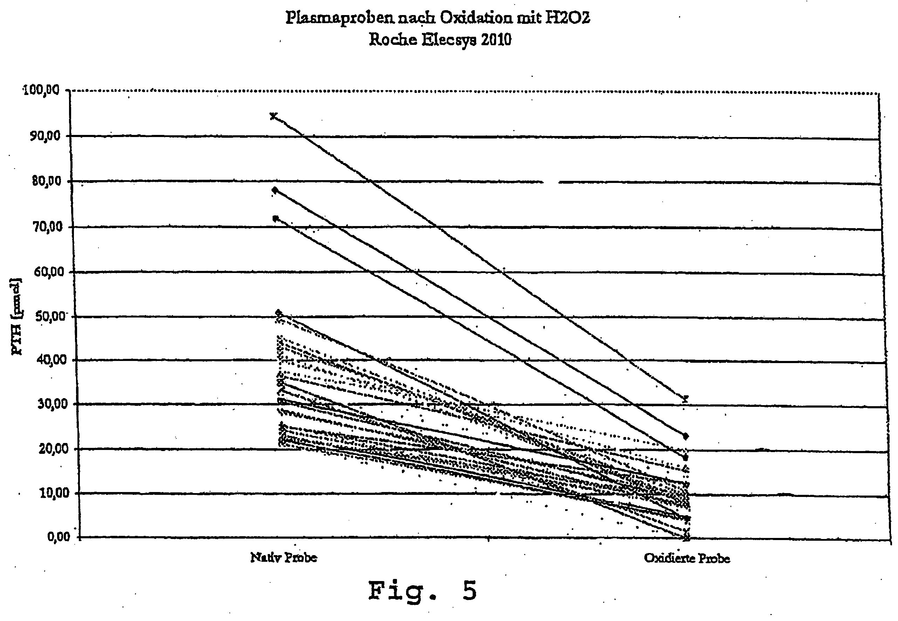

Von 20 Proben wurde je ein Aliquot im Verhältnis 1:1 (v/v) mit einer 100 nM NaBH₄-Lösung versetzt, und reduziert. Ferner wurde von 10 oxidierten Proben je ein Aliquot durch Zusatz einer 1000 nMol NaBH₄-Lösung im Verhältnis 1:1 rückreduziert. Die Proben wurden 45 Minuten bei Raumtemperatur inkubiert und die Reaktion auf Trockeneis gestoppt. Nach der Lyophilisierung wurden die Proben bis zur Bestimmung bei -20°C tiefgefroren. Die Diagramme der Figuren 5 bis 11 zeigen die Ergebnisse.

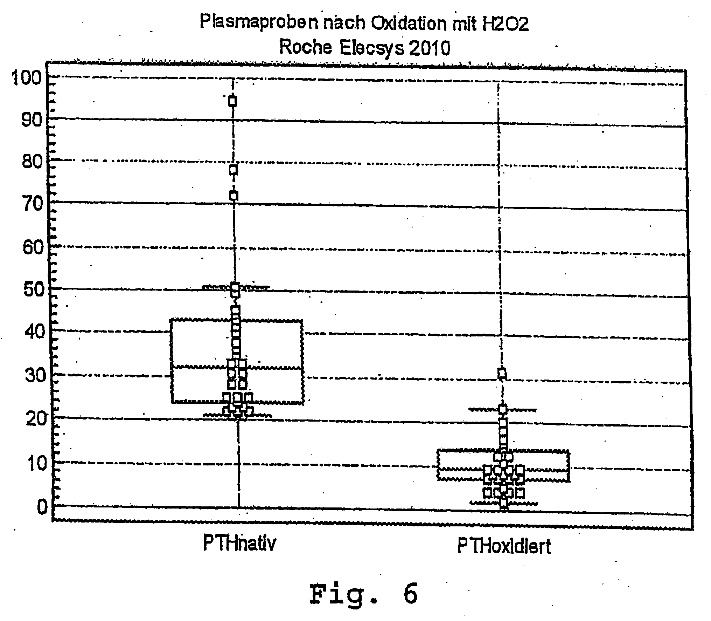

Siehe Figuren 5 und 6. Die mit dem Roche Elecsys™ 2010-PTH-Test erhobenen Messwerte für natives PTH lagen nach der Oxidation im Mittel bei 29% des Ausgangswertes. Hierbei verhielten sich die einzelnen Proben recht unterschiedlich, Die maximale Reduktion des Messsignals lag bei 5% und die minimale Reduktion des Messsignals bei 53% des Ausgangswertes. Die Ergebnisse zeigen, dass der im Roche Elecsys™ 2010-PTH-Test verwendete Fänger-Antikörper überwiegend die native, reduzierte PTH-Form erkennt.

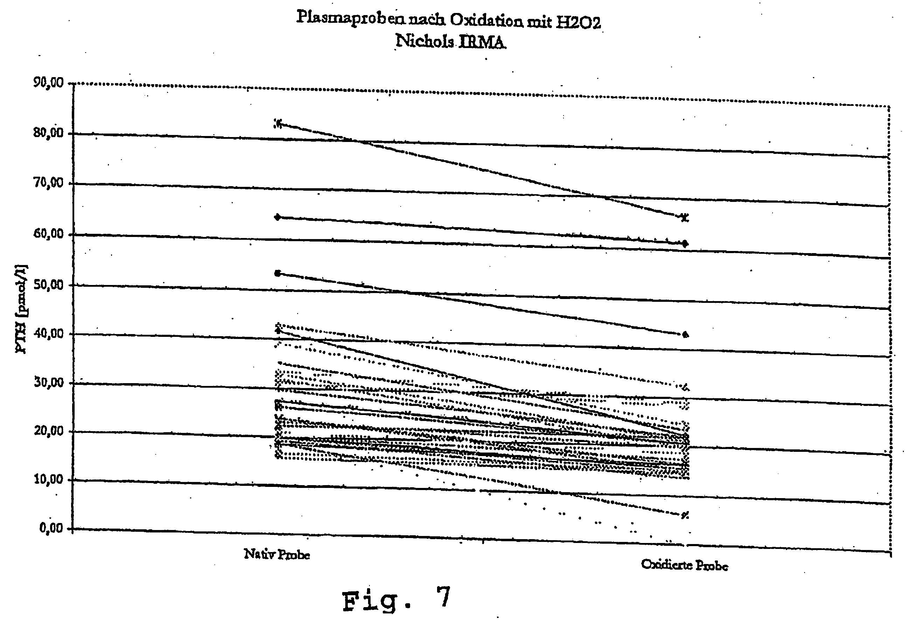

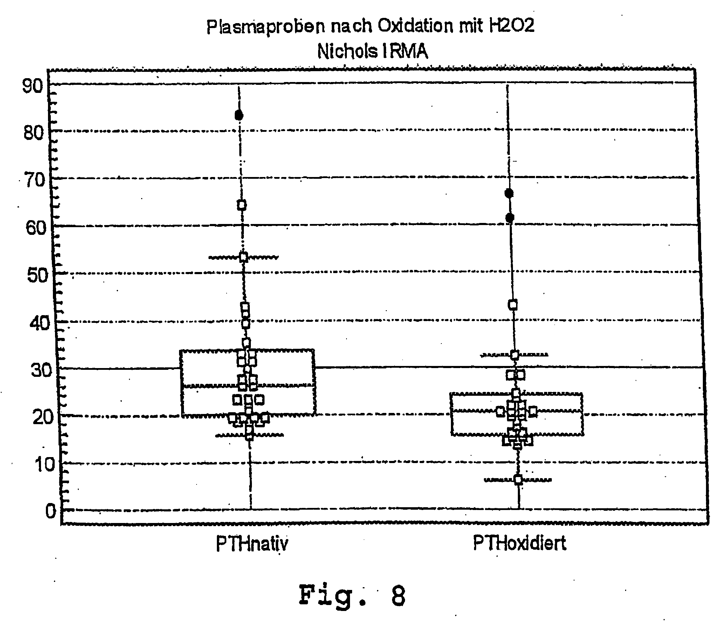

Siehe Figuren 7 und 8. Die Oxidation hatte auf die Messwerte im Nichols-IRMA deutlich geringeren Einfluss. Im Mittel reduzierte sich die Konzentration auf 76% der Ausgangswerte mit einer maximalen Reduktion auf 33% und einer minimalen Reduktion auf 98%.

Um eine partielle Zerstörung des PTH-Moleküls durch die Vorbehandlung der Proben mit H₂O₂ auszuschließen, wurden die oxidierten Proben wieder mit NABH₄ rückreduziert. Bei Messung der rückreduzierten Proben mit dem Elecsys™ 2010-PTH-Test konnten nur ca. 50% der Ausgangswerte erreicht werden. Da das Detektionssystem aber auf einer elektrochemischen Lumineszenzreaktion basiert ist, störte vermutlich das Reduktionsmittel die Bestimmung. In weiteren Versuchen war eine Störung der Signalmessung im Elecsys-System auch mit anderen Antioxidantien festzustellen. Vermutlich wurde aber auch ein Teil der Methionine zum Sulfon oxidiert, was nicht rück-reduziert werden kann.

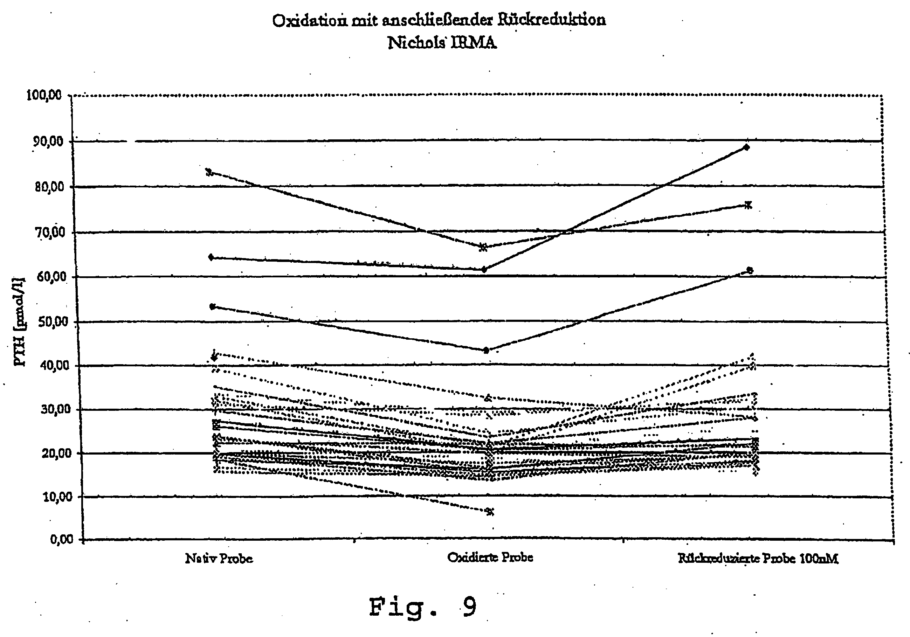

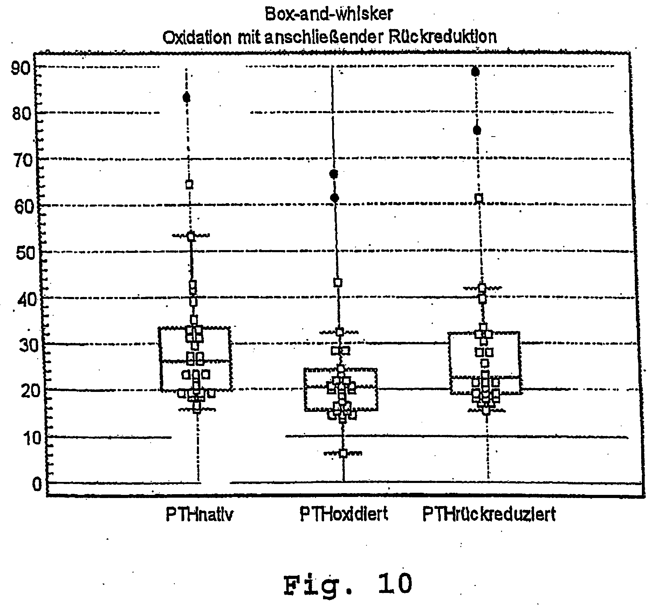

Figuren 9 und 10 zeigen die Ergebnisse im parallelen Versuch mit dem Nichols-IRMA Die Resultate belegen, dass die Oxidation bzw. Reduktion des Peptides die immunologische Aktivität nicht beeinflusst. Nach Oxidation und entsprechender Rückreduktion wurden weitgehend die nativen Ausgangskonzentrationen erreicht. Die geringen Unterschiede in der Wiederfindung nach der Rückreduktion beruhen vermutlich auf unterschiedlichen (Oxidations-)-Ausgangszuständen des Peptids.

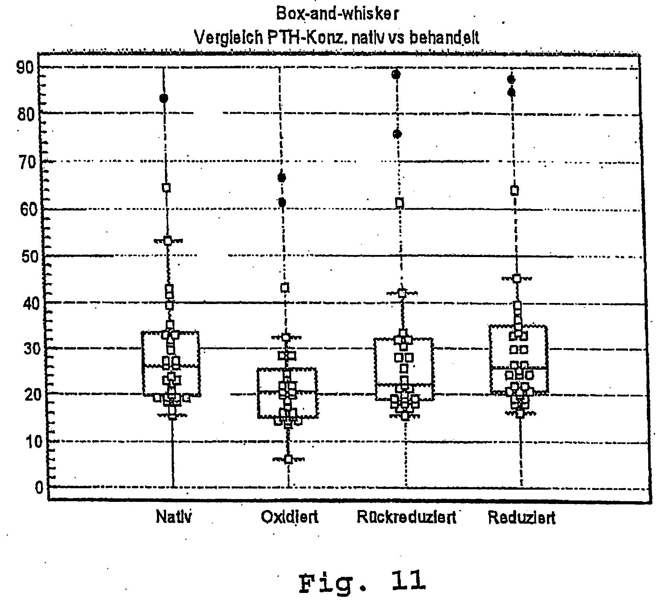

Figur 11 zeigt die Ergebnisse zur alleinigen Reduktion von nativem; PTH. Die alleinige Reduktion führte zu einer geringfügig erhöhten Wiederfindung {plus 4%) des nativen Ausgangswertes. Die im Nichols-Test eingesetzten polyklonalen Antikörper mit ihrer hohen Affinität zur oxidierten Form des Peptides verhindern wahrscheinlich eine messtechnisch fassbare, akzentuiertem Ausprägung unterschiedlicher PTH-Konzentrationen vor und nach Reduktion des Peptids.

Die Daten belegen eindeutig den Einfluss einer Oxidation auf die, mit verschiedenen Tests gemessene, Konzentration von Intakt-PTH, Natives bzw. oxidiertes PTH zeigen deutliche Unterschiede in ihrer biologischen Aktivität an verschiedenen Zielorganen. Besonders Dialysepatienten sind *offenbar* einem hohen oxidativen Stress ausgesetzt und die Vermutung liegt nahe, dass freie reaktive Sauerstoffspezies das Parathormon durch Oxidation der Methionine in seiner Struktur und somit die biologische Aktivität signifikant verändern. So leiden Dialysepatienten oftmals an Hyperhomocysteinemie, ein bekannter Indikator für Oxidationsstress, und einen gestörten Homocystein-Metabolismus (Durand P. et al. Laboratory Investigation 2001, 81(5), 645-672). Für eine Oxidation anfällig sind im Parathormon vor allem die Methionine an Position 8 und 18. Die Oxidation der Methionine an Position 8 und 18 zum Methionylsulfoxid ist vermutlich biologisch reversibel wegen der in den Geweben vorkommende Methionylsulfoxid-Reduktase (Vogt W., Free Radical Biology & Medicine, 1995, 18(1), 93-105). Sie führt sicher zu einer Verlust der physiologischen Wirkung (Galceran et al, Endocrinology, 1984, 115(6), 2375-2378; Horiuchi N. J. Bone Miner. Res., 1988, 3(3), 353-358), Zull JE et al., J, Biol. Chem., 1990, 265(10), 5671-5676; O'Riordan JLH et al., J. Endocr., 1974, 63, 117-124). Die zurzeit zur Verfügung stehenden Tests können somit nicht differenziert biologisch aktives PTH von inaktivem PTH unterscheiden,

### Beispiel 4 Selektive Blockierung der N-terminalen Bindungsstelle von oxidiertem PTH durch Zusatz von monoklonalen Antikörpern gegen oxidiertes PTH

Es wurde ein monoklonaler Antikörper gegen oxidiertes PTH gemäß Logue FC et al (Annu. Clin. Biochem. 1991, 28, 160-166; J. Immunol. Methods, 1991, 137, 156-166) eingesetzt. Dieser wurde kurz gesagt erhalten, indem synthetisches hPTH(1-34) durch Zusatz von Wasserstoffperoxid (30%) oxidiert und dann DA-Ratten damit immunisiert wurden, Es kann auch eine Immunisierung mit synthetischen PTH-Fragmenten (1-37), die an Position 8 bzw. 18 ein Methionylsulfoxid enthalten, erfolgen, Die Fusion der Rattenmilzzellen mit der Maus-Myelomline X63Ag 8.653 lieferte dann verschiedene monoklonale Antikörper, die auf ihre Eigenschaft zur Bindung und Maskierung von oxidiertem PTH in einem Immunoassay getestet wurden,

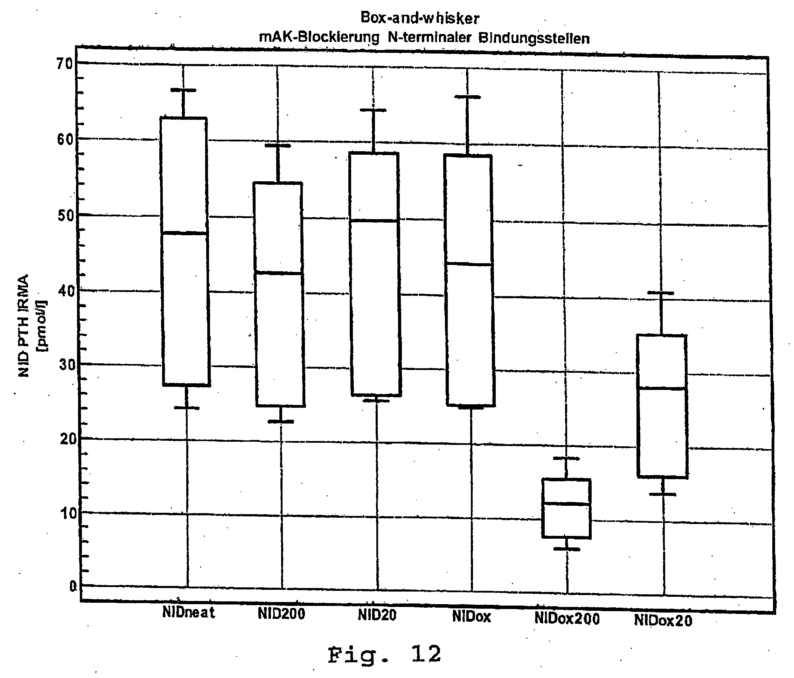

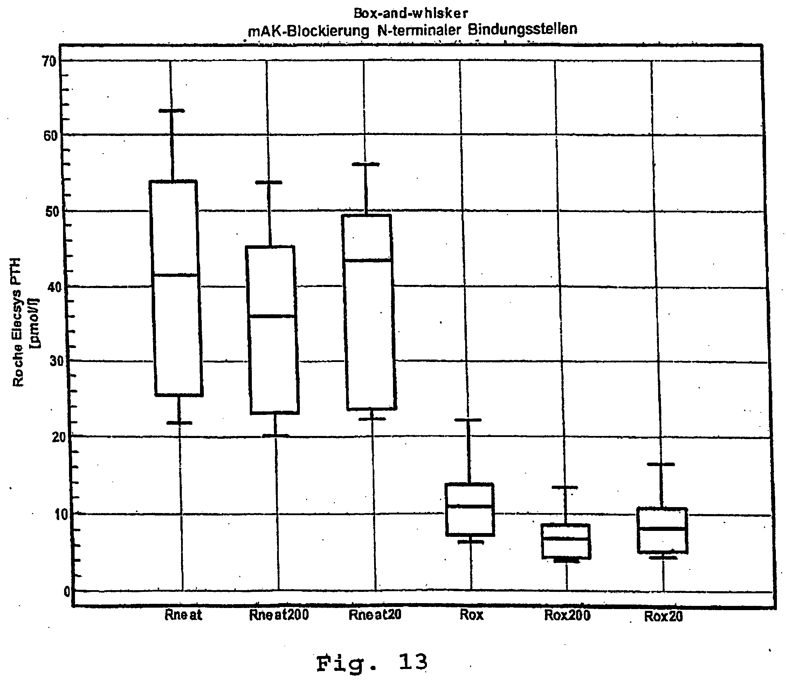

Figuren 12 und 13 zeigen Eliminationsversuche mit einem monoklonalen Antikörper mAK-hPTH(1-37)-metox-8/18 (3B3), der ein Epitop mit PTH-Methionylsulfoxid erkannte, Dieser Antikörper wurde zu Vergleichszwecken in einen herkömmlichen PTH-IRMA von Nichols Institute und im Elecsys™ 2010 PTH *der* Firma Roche eingesetzt.

Es wurden hierzu zehn EDTA-Plasmapools mit mittleren PTH-Werten im Hoch konzentrationsbereich (Mittelwert + SD: NID 46±17 pMol/L; Roche 41+14 pMol/L) hergestellt und für eine Blockierung der N-terminalen Bindungsstelle mit dem oben beschriebenen monoklonalen Antikörper gegen oxidiertes PTH versetz. Hierzu wurden die Proben mit dem einer Lösung des monoklonalen Antikörpers so vermischt, dass die Endkonzentration in der Probe bezüglich des monoklonalen Antikörpers bei 200 ng/ml bzw. 20 ng/ml lag. Die Probe wurde eine Stunde bei Raumtemperatur mit dem Maskierungsantikörper gegen oxidiertes PTH vorinkubiert und dann in herkömmlicher Weise mit dem jeweiligen Testbesteck von Roche bzw. Nichols bestimmt. Die Ergebnisse sind in Figuren 12 und 13 dargestellt.

Hierbei steht NID und R für Nichols- bzw, Roche-Test *und "neat" für* unversetzte Probe, 200 für den Zusatz von *200* ng/ml mAK-hPTH(1-37)-metox-8/18 (3B3)-Maskierungs-Antikörper, 20 für *den Zusatz von 20 ng*/*ml* Maskierungsantikörper, Rox bzw. NIDox für eine Wasserstoffperoxid - oxidierte Probe gemäß Beispiel 3 und 200 bzw. 20 für den angegebenen Zusatz von Maskierungsantikörper.

Die Ergebnisse zeigen, dass der PTH-Assay von Nichols-Institute nicht zwischen oxidiertinaktiven PTH und nativ-aktivem PTH unterscheiden kann, dass aber durch den Zusatz von Maskierungsantikörpem gegen oxidiertes PTH - selbst bei einem polyklonalen Ziegen-Antikörper gegen hPTH(1-34) - eine Antikörperbindung im N-terminalen Abschnitt und eine. Fehlbestimmung weitestgehend unterdrückt werden kann. Die Versuche zeigen ferner, dass selbst normale Poolplasmen ca. 10% oxidiertes PTH enthalten.

Der Roche-PTH-Test enthält zwar einen Konformations-Nachweisantikörper (PTH 55-64), der anscheinend zwischen oxidiertem und nativ-aktivem PTH unterscheiden kann. Dennoch werden durch den Zusatz von Maskierungsantikörper falsche Überbestimmungen, insbesondere in der unversetzten Probe, deutlich reduziert.

Zu beachten ist, dass beide Vergleichstests keine aktiven PTH-Fragmente des Typs hPTH(1-34v37) erfassen und somit immanent falsche PTH-Gehalte liefern.

## Patentansprüche

1. Testkit zur quantitativen Bestimmung des biologisch wirksamen Parathormons in einer Probe einer Körperflüssigkeit durch einen Sandwich-Immunoassay auf intakte humane Parathormon-Polypeptidketten und N-terminal intakter Fragmente hiervon, umfassend Antikörper, die an oder nächst der PTH-Rezeptor-Bindungsstruktur im N-terminalen Abschnitt des humanen Parathormons binden und Antikörper, die an ein Epitop binden, das von mindestens einer oxidierten Methioningruppe an Position 8 und/oder 18 des humanen Parathormons gebildet wird.

2. Testkit nach Anspruch 1, wobei die Antikörper gegen den N-terminalen Abschnitt 1 bis 37 des Parathormons durch Affinitätschromatographie von Antikörpern gereinigt sind, die an Methionin 8 und/oder 18 der oxidierten Parathormon-Polypeptidkette binden.

3. Testkit nach Anspruch 1 oder 2, umfassend einen Antikörper, der ein Epitop erkennt, das von den N-terminalen Aminosäuren 1 bis 3 des Parathormons gebildet wird.

4. Verwendung eines Testkits nach einem der Ansprüche 1 bis 3 zur Bestimmung des oxidativen Stresses in Dialysepatienten.

## Claims

1. Kit of parts for determining quantitatively the biologically active parathyroid hormone in a sample of the body fluid by means of a sandwich immunoassay for intact human parathyroid hormone peptide chains and N-terminally intact fragments thereof, comprising antibodies which bind at or adjacent the PTH receptor binding structure within the N-terminally region of the human parathyroid hormone and antibodies binding to an epitope, which consists at least of an oxidized methionine group at position 8 and/ or 18 of the human parathyroid hormone.

2. Kit of parts in accordance with claim 1, where in the antibodies against the N-terminal region with amino acids 1 to 37 of the parathyroid hormone have been purified by affinity, chromatography of antibodies, which bind to methionine 8 and/ or 18 of the oxidized polypeptide of the parathyroid hormone.

3. Kit of parts as claimed in claim 1 or 2, comprising an antibody, which binds to an epitope, that is formed by the N-terminal amino acids 1 to 3 of the parathyroid hormone.

4. Use of a test kit as claimed in any claim 1 to 3 for determining of the oxidative stress of patients undergoing dialysis treatment.

## Revendications

1. Kit de test pour la détermination quantitative de la parathormone biologiquement active dans un échantillon de fluide corporel par un dosage immunologique de type sandwich de chaînes polypeptidiques intactes de parathormone humaine et de leurs fragments intacts sur les extrémités N-terminales, comprenant des anticorps qui se lient à ou à côté de la structure de liaison du récepteur PTH dans la partie des extrémités N-terminale de la parathormone humaine et des anticorps qui se lient à un épitope qui est formé par au moins un groupe oxydé de méthionine aux positions 8 et/ou 18 de la parathormone humaine.

2. Kit de test selon la revendication 1, dans lequel les anticorps contre la partie 1 à 37 de l'extrémité N-terminale de la parathormone sont des anticorps qui se lient à la méthionine 8 et/ou 18 de la chaîne oxydée de polypeptides de parathormone purifiés par chromatographie d'affinité.

3. Kit de test selon la revendication 1 ou 2, comprenant un anticorps qui reconnaît un épitope qui est formé par les acides aminés 1 à 3 de l'extrémité N-terminale de la parathormone.

4. Utilisation d'un kit de test selon l'une des revendications 1 à 3 pour la détermination du stress oxydatif de patients dialysés.
